# EUROPEAN PATENT APPLICATION

(11) **EP 2 639 220 A1**
(43) Date of publication of application: **18.09.2013**
(21) Application number: 12382097.9
(22) Date of filing: 15.03.2012
(51) Int. Cl.: C07D 209/08, C07D 401/12, C07D 407/04, C07D 409/14, A61K 31/404, A61P 31/04

(54) **Indole derivatives and their use as antibiotics**

(71) Applicant: Omnia Molecular, S. L., 08028 Barcelona (ES)
(72) Inventor: Serra Comas, Maria Del Carmen, 08034 Barcelona (ES); Rey Puiggròs, Oscar, 08028 Barcelona (ES); Harrak Serifi, Youssef, 08034 Barcelona (ES); Llebaria Soldevila, Amadeo, 08034 Barcelona (ES); Vidal Montull, David, 08018 Barcelona (ES); Mestres López, Jordi, 08003 Barcelona (ES); Yu, Hua, 610041 Chengdu (CN); Chen, Li, 610041 Chengdu (CN); Li, Zehua, 610041 Chengdu (CN); Fan, Lei, 610041 Chengdu (CN)
(74) Representative: ZBM Patents

(57) **Abstract**

Compound of formula (I) or a pharmaceutically acceptable salt thereof, or a stereoisomer thereof or a mixture of stereoisomers, which are useful as antibiotics. The invention also provides processes for the preparation of the compounds of formula (I) and intermediates, their compositions, their use as medicaments and methods for treating bacterial infections.

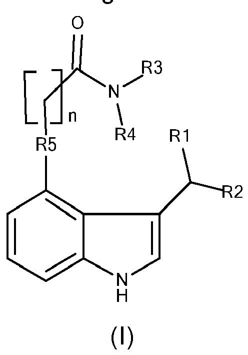

## Description

The present invention is related to the fields of chemistry and medicine. More particularly, the present invention refers to new 3,4-substituted indole compounds which are useful as antibiotics.

### BACKGROUND ART

Pathogenic bacteria that have become resistant to antibiotic drug therapy are an increasing public health problem. Part of the problem is that infective bacteria are remarkably able to develop resistance to antibacterial drugs. A larger part of the problem is due to the use and misuse of existing antibiotics in human and veterinary medicine and in agriculture.

There is substantial concern worldwide with the mounting prevalence of infections caused by multidrug-resistant bacteria, including methicillin-resistant *Staphylococcus aureus,* vancomycin-resistant *Enterococci, Mycoplasma pneumoniae*, and certain Gram-negative bacteria such as *Pseudomonas aeruginosa, Acinetobacter baumanii*, and *Klebsiella pneumoniae.* Such infections are extremely difficult to control, and are a prevalent cause of disease and mortality. Conventional antibiotics typically interact with one or more specific target protein or receptor, and genetic resistance appears at a rate that depends on many factors, such as the number of targets.

A number of structural classes of compounds are known with antibacterial activity.The most important classes of antibacterials are β-lactams, macrolides, lincosamides, aminoglycosides, tetracyclines, polypeptides, and sulfonamides.

Further, there are many antibacterial compounds not belonging to any of the cited common classes. For example, some compounds comprising an indole moiety have been synthesized and disclosed as showing antibiotic properties. Thus, WO1998057931 discloses some quinoline-3-indole derivatives as antibiotics. Although it is mentioned in this document that the position 3 of the indole moiety of these derivatives can be substituted by a quinoline group, and that the position 4 can be substituted by alkyl, alkoxyl or amino, no specific compounds with this particular substitution are disclosed.

Other indole derivatives are known in the art for having antibiotic activity. For instance, Pereira et al. "Synthesis and antimicrobial activities of five membered ring heterocycles coupled to indole moieties", The Journal of Antibiotics, 1996, Vol. 49, n. 4, pp 380-385, describe oxazolone-3-indoles as showing antimicrobial activity on a selection of bacterial species.

From what it is known in the art it is derived that there is still the need of finding new and effective antibiotic compounds. The older and more used antibiotics often become either ineffective or significantly less effective against the pathogens due to the great ability of bacteria to generate resistance to existing antibiotics. Effective antibiotics are therefore in constant demand by the field of medicine to replace the older and more used antibiotics and to overcome the existing resistance mechanisms. Because of the above mentioned long felt need in this art for potent and effective antibiotics, even finding small improvements or alternatives can sometimes be very significant.

### SUMMARY OF THE INVENTION

New 3,4-substituted indole compounds have shown to be active as inhibitors of bacterial tryptophanyl-tRNA synthetase (WRS). Thus, the new 3,4-substituted indole compounds have been found to be useful as antibiotic agents.

Thus, according to a first aspect, the present invention provides a compound of formula (I) or a pharmaceutically acceptable salt thereof, or a stereoisomer thereof or a mixture of stereoisomers, 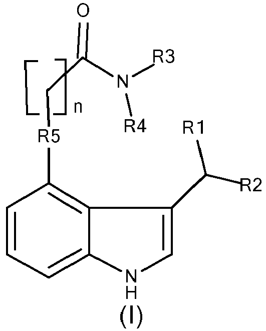 where R1 and R2 are independently (C1-C4)alkyl, or alternatively, R1 and R2 together with the C atom to which are attached form a ring of 4-7 members, each member independently selected from the group consisting of C, N, O, S, CH, CH₂, and NH, the ring being saturated or partially unsaturated and optionally substituted by one to three radicals selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, -NRa-C(O)-NRaRb, and benzyl; R3 and R4 are independently selected from the group consisting of hydrogen; (C1-C4)alkyl optionally substituted by one to three radicals selected from -OH, a ring of 4-6 members, each member independently selected from the group consisting of C, N, O, S, CH, CH₂, and NH, the ring being saturated, partially unsaturated, or aromatic and optionally substituted by one to three radicals selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, -NRa-C(O)-NRaRb; -(C1-C4)alkyl-C(O)-(C1-C8)alkyl; -(C1-C4)alkyl-COO-(C1-C8)alkyl; -(C1-C4)alkyl-NRaRb_{;} a ring of 3-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and -NRa-C(O)-NRaRb, and a ring of 5-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and -NRa-C(O)-NRaRb; or alternatively, R3 and R4 together with the N atom to which are attached form a ring of 5-6 members, each member independently selected from C, N, O, S, CH, CH₂, and NH, the ring being saturated or partially unsaturated, optionally substituted with by one to three radicals selected from the group consisting of: halogen; -OH; -(C1-C4)alkyl optionally substituted by -OH; (C1-C4)alkoxyl; -C(O)-(C1-C4)alkyl; -(C1-C4)alkyl-C(O)-(C1-C4)alkyl; -COO-(C1-C4)alkyl; -(C1-C4)alkyl-COO-(C1-C4)alkyl; -NRaRb; -(C1-C4)alkyl-NRaRb; -C(O)-NRaRb; -NRa-C(O)-(C1-C4)alkyl; -NRa-C(O)-NRaRb; and a ring of 3-6 members, each member independently selected from C, N, O, S, CH, CH₂, and NH, the ring being saturated or partially unsaturated. R5 is an atom selected from the group consisting of N, C, and O; n is an integer from 1 to 3; and Ra and Rb are independently selected from the group consisting of H and (C1-C4)alkyl.

As a second aspect, the invention provides a process for the preparation of a compound of formula (I) as defined above, comprising the step of reacting a compound of formula (II), 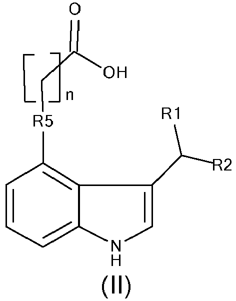 wherein R1, R2, R5, and n are as defined above; with an amine of formula R3R4NH, wherein R3 and R4 are as defined above.

According to its third aspect, the invention provides intermediate compounds of formula (II).

Further, as a fourth aspect of the invention, intermediate compounds of formula (III) are provided, 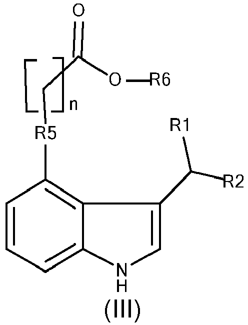 where R6 is a (C1-C4)alkyl, and R1, R2, R5, and n are as defined above.

As mentioned, the compounds of formula (I) are useful for treating bacterial infections.

Thus, another aspect of the invention relates to the compounds of formula (I) for use as a medicament.

A further aspect of the invention relates to the compounds of formula (I) for use in treatment of bacterial infections.

Finally, the last aspect of the invention relates to a pharmaceutical composition comprising a therapeutically effective amount of the compounds of formula (I), together with adequate amounts of pharmaceutical excipients or carriers.

### DETAILED DESCRIPTION OF THE INVENTION

The terms "(C1-C2)alkyl", "(C1-C3)alkyl", "(C1-C4)alkyl", and "(C1-C8)alkyl" as used herein refers to an optionally substituted saturated branched or linear hydrocarbon chains with 1 to 2, 1 to 3, 1 to 4, or 1 to 8 carbon atoms, respectively.

The term "cyclo-(C3-C6)alkyl" as used herein refers to an optionally substituted saturated cyclic hydrocarbon chain with 3 to 6 carbon atoms, for instance cyclopropyl, cyclobutyl, cyclopentyl, and cyclohexyl.

The term "room temperature" as used herein refers to a temperature ranging from 20 to 25 °C.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

As mentioned above, an aspect of the present invention is the provision of compounds of formula (I) a salt or its stereoisomers and their mixtures thereof as defined above.

Any pharmaceutically acceptable salt of the compounds of formula (I) can be used for the purposes of the invention.

The term "pharmaceutically acceptable salts" used herein encompasses any salt formed from pharmaceutically acceptable non-toxic acids including inorganic or organic acids. There is no limitation regarding the salts, except that if used for therapeutic purposes, they must be pharmaceutically acceptable.

As some of the compounds of formula (I) are basic compounds, salts may be prepared form pharmaceutically acceptable non-toxic acids, including inorganic and organic acids. Such acids include for instance acetic, benzenesulfonic, benzoic, camphorsulfonic, citric, ethansulfonic, fumaric, gluconic, glutamic, hydrobromic, hydrochloric, lactic, maleic, malic, mandelic, methanesulfonic, phosphoric, succinic, sulfuric, tartaric, p-toluensulfonic acid, and the like.

The term "compounds of formula (I)" used herein encompasses the compounds of formula (I) or a pharmaceutically acceptable salt thereof, or a stereoisomer thereof or a mixture of stereoisomers, as defined in the first aspect of the invention.

The compounds of formula (I) may be in crystalline form either as free solvation compounds or as solvates (e.g. hydrates) and it is intended that both forms are within the scope of the present invention. Methods of solvation are generally known within the art.

Compounds of formula (I) may have asymmetric centers and, therefore, exist in different enantiomeric or diastereomeric forms. All single optical isomers and stereoisomers of the compounds referred to herein, and mixtures thereof, are considered within the scope of the present invention. Thus, any given compound referred to herein is intended to represent any one of a racemate, one or more enantiomeric or diastereomeric forms, one or more atropoisomeric forms, and mixtures thereof.

In a preferred embodiment of the invention, R5 is an oxygen atom and n is 1 in compounds of formula (I).

In a further preferred embodiment, R1 and R2 are independently (C1-C4)alkyl. More preferably, R1 and R2 are independently selected from the group consisting of (C1-C2)alkyl. In an alternative further preferred embodiment, R1 and R2 together with the carbon atom to which are attached form a ring of 4-6 members, each member independently selected from the group consisting of C, N, O, S, CH, CH₂, and NH, the ring being saturated or partially unsaturated. More preferably, R1 and R2 together with the C atom to which are attached, form a ring selected from the group consisting of cyclobutyl, cyclopentyl, cyclohexyl, oxanyl, and tetrahydrothienyl.

In a particular embodiment, the invention provides compounds of formula (I) as defined in the first aspect of the invention where R1 and R2 are independently (C1-C4)alkyl. In an alternative particular embodiment, R1 and R2 together with the C atom to which are attached form a ring of 4-6 members, each member independently selected from the group consisting of C, N, O, S, CH, CH₂, and NH, the ring being saturated or partially unsaturated.

In a preferred embodiment of the invention, R3 and R4 are independently selected from the group consisting of hydrogen; (C1-C4)alkyl optionally substituted by one to three radicals selected from -OH, a ring of 5-6 members, each member independently selected from the group consisting of C, N, O, S, CH, CH₂, and NH, the ring being saturated, partially unsaturated, or aromatic; -CH₂-COO-(C1-C4)alkyl; and a ring of 3-6 members, each member independently selected from C, N, O, CH, CH₂, and NH, the ring being saturated, partially unsaturated, or aromatic, optionally substituted by one to three radicals selected from the group consisting of: -OH, and -(C1-C4)alkyl; or alternatively, R3 and R4 together, with the N atom within, form a ring of 5-6 members, each member independently selected from C, N, O, CH, CH₂, and NH, the ring being saturated or partially unsaturated, optionally substituted by one to three radicals selected from the group consisting of: -OH; -(C1-C4)alkyl optionally substituted by -OH; -C(O)-(C1-C4)alkyl; -COO-(C1-C4)alkyl; and a ring of 5-6 members, each member independently selected from C, N, O, S, CH, CH₂, and NH, the ring being saturated.

Another preferred embodiment of the invention relates to a compound of formula (I) where R3 and R4 are independently selected from the group consisting of hydrogen; (C1-C4)alkyl optionally substituted by one to three radicals selected from -OH, phenyl, and pyridinyl; -CH₂-COO-(C1-C4)alkyl; pyridinyl; cyclopropyl, and cyclohexyl, optionally substituted with -OH. In an alternative preferred embodiment R3 and R4 together with the N atom to which are attached form a ring selected from the group consisting of piperazine, morpholine, and piperidine, optionally substituted by -C(O)-(C1-C4)alkyl, -COO-(C1-C4)alkyl, and pyrrolidinyl.

In another preferred embodiment compounds of formula (I) are those where R3 and R4 are independently selected from the group consisting of hydrogen; (C1-C2)alkyl optionally being substituted by one to three radicals selected from -OH, phenyl and pyridinyl; -CH₂-COO-(C1-C3)alkyl; and pyridinyl; R3 and R4 together with the N atom to which are attached, form a group selected from the group consisting of piperazine, and morpholine; optionally substituted by -C(O)-(C1-C2)alkyl.

In a particular embodiment of the invention compounds of formula (I) are those where R1 and R2 are independently (C1-C4)alkyl, or, in an alternative particular embodiment, R1 and R2 together with the C atom to which are attached form a ring of 4-6 members, each member independently selected from the group consisting of C, N, O, S, CH, CH₂, and NH, the ring being saturated or partially unsaturated; and R3 and R4 are independently selected from the group consisting of hydrogen; (C1-C4)alkyl optionally substituted by one to three radicals selected from -OH, phenyl and pyridinyl; -CH₂-COO-(C1-C4)alkyl, pyridinyl, and cyclo-(C3-C6)alkyl; optionally substituted with -OH; R3 and R4 together with the N atom to which are attached form a ring selected from the group consisting of piperazine,

morpholine, and piperidine; optionally substituted by -C(O)-(C1-C4)alkyl, -COO-(C1-C4)alkyl, and pyrrolidinyl. More particularly, R5 is an oxygen atom and n is 1.

In another particular embodiment of the invention, compounds of formula (I) are those where R1 and R2 are independently (C1-C4)alkyl, or, in an alternative particular embodiment, R1 and R2 together with the C atom to which are attached form a ring of 4-6 members, each member independently selected from the group consisting of C, N, O, S, CH, CH₂, and NH, the ring being saturated or partially unsaturated; and R3 and R4 are independently selected from the group consisting of hydrogen; (C1-C2)alkyl optionally being substituted by one to three radicals selected from -OH, phenyl and pyridinyl; -CH₂-COO-(C1-C3)alkyl; and pyridinyl; R3 and R4 together with the N atom to which are attached, form a group selected from the group consisting of piperazine, and morpholine; optionally substituted by -C(O)-(C1-C2)alkyl. More particularly, R5 is an oxygen atom and n is 1.

In a further particular embodiment of the invention, compounds of formula (I) are those where R1 and R2 are independently selected from the group consisting of (C1-C2)alkyl; R1 and R2 together with the C atom to which are attached, form a ring selected from the group consisting of cyclobutyl, cyclopentyl, cyclohexyl, oxanyl, and tetrahydrothienyl; and R3 and R4 are independently selected from the group consisting of hydrogen; (C1-C2)alkyl optionally being substituted by one to three radicals selected from -OH, phenyl and pyridinyl; -CH₂-COO-(C1-C3)alkyl; and pyridinyl; R3 and R4 together with the N atom to which are attached, form a group selected from the group consisting of piperazine, and morpholine; optionally substituted by -C(O)-(C1-C2)alkyl. More particularly, R5 is an oxygen atom and n is 1.

In another particular embodiment the compounds of formula (I) are the following ones:

**Table 1**

| | **Compound Name** |
|---|---|
| (I)-1 | N-(pyridin-3-ylmethyl)-2-((3-(tetrahydrothiophen-3-yl)-1H-indol-4-yl)oxy)acetamide |
| (I)-2 | N-(I-1-phenylethyl)-2-((3-(tetrahydrothiophen-3-yl)-1H-indol-4-yl)oxy)acetamide |
| (I)-3 | N-(pyridin-4-yl)-2-((3-(tetrahydrothiophen-3-yl)-1H-indol-4-yl)oxy)acetamide |
| (I)-4 | N-(pyridin-3-yl)-2-((3-(tetrahydrothiophen-3-yl)-1H-indol-4-yl)oxy)acetamide |
| (I)-5 | N-(pyridin-2-ylmethyl)-2-((3-(tetrahydrothiophen-3-yl)-1H-indol-4-yl)oxy)acetamide |
| (I)-6 | N-benzyl-2-((3-(tetrahydrothiophen-3-yl)-1H-indol-4-yl)oxy)acetamide |
| (I)-7 | N-(pyridin-4-ylmethyl)-2-((3-(tetrahydrothiophen-3-yl)-1H-indol-4-yl)oxy)acetamide |
| (I)-8 | N-((S)-1-phenylethyl)-2-((3-(tetrahydrothiophen-3-yl)-1H-indol-4-yl)oxy)acetamide |
| (I)-9 | propyl 2-(2-((3-(tetrahydrothiophen-3-yl)-1H-indol-4-yl)oxy)acetamido)acetate |
| (I)-10 | N-cyclopropyl-2-((3-(tetrahydrothiophen-3-yl)-1H-indol-4-yl)oxy)acetamide |
| (I)-11 | N-isopropyl-2-((3-(tetrahydrothiophen-3-yl)-1H-indol-4-yl)oxy)acetamide |
| (I)-12 | N-(1-hydroxybutan-2-yl)-2-((3-(tetrahydrothiophen-3-yl)-1H-indol-4-yl)oxy)acetamide |
| (I)-13 | isopropyl 2-(2-((3-(tetrahydrothiophen-3-yl)-1H-indol-4-yl)oxy)acetamido)acetate |
| (I)-14 | N-(4-hydroxycyclohexyl)-2-((3-(tetrahydrothiophen-3-yl)-1H-indol-4-yl)oxy)acetamide |
| (I)-15 | N-(2-hydroxypropyl)-2-((3-(tetrahydrothiophen-3-yl)-1H-indol-4-yl)oxy)acetamide |
| (I)-16 | methyl 2-(2-((3-(tetrahydrothiophen-3-yl)-1H-indol-4-yl)oxy)acetamido)acetate |
| (I)-17 | 2-((3-(pentan-3-yl)-1H-indol-4-yl)oxy)-N-(pyridin-4-ylmethyl)acetamide |
| (I)-18 | 2-((3-(pentan-3-yl)-1H-indol-4-yl)oxy)-N-(pyridin-3-ylmethyl)acetamide |
| (I)-19 | 2-((3-(pentan-3-yl)-1H-indol-4-yl)oxy)-N-(pyridin-3-yl)acetamide |
| (I)-20 | 2-((3-(pentan-3-yl)-1H-indol-4-yl)oxy)-N-(pyridin-2-ylmethyl)acetamide |
| (I)-21 | 1-(4-acetylpiperazin-1-yl)-2-((3-(pentan-3-yl)-1H-indol-4-yl)oxy)ethanone |
| (I)-22 | methyl 2-(2-((3-(pentan-3-yl)-1H-indol-4-yl)oxy)acetamido)acetate |
| (I)-23 | 1-morpholino-2-((3-(pentan-3-yl)-1H-indol-4-yl)oxy)ethanone |
| (I)-24 | propyl 2-(2-((3-(pentan-3-yl)-1H-indol-4-yl)oxy)acetamido)acetate |
| (I)-25 | N-(1-hydroxybutan-2-yl)-2-((3-(pentan-3-yl)-1H-indol-4-yl)oxy)acetamide |
| (I)-26 | N-methyl-2-((3-(pentan-3-yl)-1H-indol-4-yl)oxy)acetamide |
| (I)-27 | N-cyclopropyl-2-((3-(pentan-3-yl)-1H-indol-4-yl)oxy)acetamide |
| (I)-28 | N-(2-hydroxyethyl)-2-((3-(pentan-3-yl)-1H-indol-4-yl)oxy)acetamide |
| (I)-29 | N-(4-hydroxycyclohexyl)-2-((3-(pentan-3-yl)-1H-indol-4-yl)oxy)acetamide |
| (I)-30 | 1-(4-hydroxypiperidin-1-yl)-2-((3-(pentan-3-yl)-1H-indol-4-yl)oxy)ethanone |
| (I)-31 | (R)-N-(1-phenylethyl)-2-((3-(tetrahydro-2H-pyran-4-yl)-1H-indol-4-yl)oxy)acetamide |
| (I)-32 | (S)-N-(1-phenylethyl)-2-((3-(tetrahydro-2H-pyran-4-yl)-1H-indol-4-yl)oxy)acetamide |
| (I)-33 | N-benzyl-2-((3-(tetrahydro-2H-pyran-4-yl)-1H-indol-4-yl)oxy)acetamide |
| (I)-34 | N-(pyridin-2-ylmethyl)-2-((3-(tetrahydro-2H-pyran-4-yl)-1H-indol-4-yl)oxy)acetamide |
| (I)-35 | N-(pyridin-3-ylmethyl)-2-((3-(tetrahydro-2H-pyran-4-yl)-1H-indol-4-yl)oxy)acetamide |
| (I)-36 | N-(pyridin-4-ylmethyl)-2-((3-(tetrahydro-2H-pyran-4-yl)-1H-indol-4-yl)oxy)acetamide |
| (I)-37 | N-(pyridin-3-yl)-2-((3-(tetrahydro-2H-pyran-4-yl)-1H-indol-4-yl)oxy)acetamide |
| (I)-38 | isopropyl 2-(2-((3-(tetrahydro-2H-pyran-4-yl)-1H-indol-4-yl)oxy)acetamido)acetate |
| (I)-39 | 1-morpholino-2-((3-(tetrahydro-2H-pyran-4-yl)-1H-indol-4-yl)oxy)ethan one |
| (I)-40 | Methyl 2-(2-((3-(tetrahydro-2H-pyran-4-yl)-1H-indol-4-yl)oxy)acetamido)acetate |
| (I)-41 | 2-((3-cyclobutyl-1H-indol-4-yl)oxy)-N-(pyridin-4-ylmethyl)acetamide |
| (I)-42 | 2-((3-cyclobutyl-1H-indol-4-yl)oxy)-N-(pyridin-2-ylmethyl)acetamide |
| (I)-43 | 1-(4-acetylpiperazin-1-yl)-2-((3-cyclobutyl-1H-indol-4-yl)oxy)ethanone |
| (I)-44 | 2-((3-cyclohexyl-1H-indol-4-yl)oxy)-N-(pyridin-2-ylmethyl)acetamide |
| (I)-45 | 2-((3-cyclohexyl-1H-indol-4-yl)oxy)-N-(2-hydroxyethyl)acetamide |
| (I)-46 | methyl 2-(2-((3-cyclohexyl-1H-indol-4-yl)oxy)acetamido)acetate |
| (I)-47 | 2-((3-cyclopentyl-1H-indol-4-yl)oxy)-N-(pyridin-3-ylmethyl)acetamide |
| (I)-48 | 1-(4-acetylpiperazin-1-yl)-2-((3-cyclopentyl-1H-indol-4-yl)oxy)ethanone |
| (I)-49 | 2-((3-cyclopentyl-1H-indol-4-yl)oxy)-N-(2-hydroxyethyl)acetamide |
| (I)-50 | 2-((3-cyclopentyl-1H-indol-4-yl)oxy)-1-morpholinoethanone |
| (I)-51 | 2-((3-cyclopentyl-1H-indol-4-yl)oxy)-N-cyclopropylacetamide |
| (I)-52 | 2-((3-cyclopentyl-1H-indol-4-yl)oxy)-N-methylacetamide |
| (I)-53 | N-benzyl-2-((3-cyclopentyl-1H-indol-4-yl)oxy)acetamide |
| (I)-54 | 2-((3-cyclopentyl-1H-indol-4-yl)oxy)-N-(4-hydroxycyclohexyl)acetamide |
| (I)-55 | 2-((3-cyclopentyl-1H-indol-4-yl)oxy)-N-(1-hydroxybutan-2-yl)acetamide |
| (I)-56 | propyl 2-(2-((3-cyclopentyl-1H-indol-4-yl)oxy)acetamido)acetate |
| (I)-57 | isopropyl 2-(2-((3-cyclopentyl-1H-indol-4-yl)oxy)acetamido)acetate |
| (I)-58 | 2-((3-isopropyl-1H-indol-4-yl)oxy)-N-(pyridin-4-ylmethyl)acetamide |
| (I)-59 | 2-((3-isopropyl-1H-indol-4-yl)oxy)-N-(pyridin-4-yl)acetamide |
| (I)-60 | 2-((3-isopropyl-1H-indol-4-yl)oxy)-N-(pyridin-3-ylmethyl)acetamide |
| (I)-61 | 2-((3-isopropyl-1H-indol-4-yl)oxy)-N-(pyridin-3-yl)acetamide |
| (I)-62 | (S)-2-((3-isopropyl-1H-indol-4-yl)oxy)-N-(1-phenylethyl)acetamide |
| (I)-63 | 2-((3-isopropyl-1H-indol-4-yl)oxy)-N-(pyridin-2-ylmethyl)acetamide |
| (I)-64 | isopropyl 2-(2-((3-isopropyl-1H-indol-4-yl)oxy)acetamido)acetate |
| (I)-65 | N-cyclopropyl-2-((3-isopropyl-1H-indol-4-yl)oxy)acetamide |
| (I)-66 | 2-((3-isopropyl-1H-indol-4-yl)oxy)-1-morpholinoethanone |
| (I)-67 | methyl 2-(2-((3-isopropyl-1H-indol-4-yl)oxy)acetamido)acetate |
| (I)-68 | ethyl 1-(2-((3-(sec-butyl)-1H-indol-4-yl)oxy)acetyl)piperidine-3-carboxylate |
| (I)-69 | 4-((3-cyclobutyl-1H-indol-4-yl)oxy)-N-cyclopropylbutanamide |
| (I)-70 | 4-((3-cyclobutyl-1H-indol-4-yl)oxy)-1-morpholinobutan-1-one |
| (I)-71 | 1-(4-hydroxypiperidin-1-yl)-4-((3-isopropyl-1H-indol-4-yl)oxy)butan-1-one |
| (I)-72 | 4-((3-cyclobutyl-1H-indol-4-yl)oxy)-1-(4-hydroxypiperidin-1-yl)butan-1-one |
| (I)-73 | 4-((3-cyclopentyl-1H-indol-4-yl)oxy)-1-(4-(pyrrolidin-1-yl)piperidin-1-yl)butan-1-one |

Furthermore, the present invention covers all possible combinations of particular and preferred groups described hereinabove.

Compounds of formula (I) may be prepared by a process comprising the step of reacting a compound of formula (II), 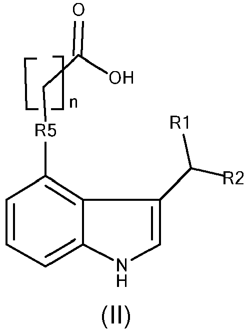 wherein R1, R2, R5, and n are as defined above for compound of formula (I); with an amine of formula R3R4NH, wherein R3 and R4 are as defined above for compound of formula (I) following known methods in the art.

Thus, the carboxilic acid (II) is treated with the amine derivative of formula R3R4NH in presence of an organic base as N,N-diisopropylethylamine (DIEA) or triethylamine in a polar organic solvent, for instance dichloromethane (DCM), dimethylformamide (DMF) or ethylacetate (EtOAc), and a suitable coupling agent, for example O-(7-azabenzotriazol-1-yl)-N,N,N',N'-tetramethyluronium hexafluorophosphate (HATU) or a combination of 1-(3-dimethylaminopropyl)-3-ethylcarbodiimide (EDC) and 1-hydroxybenzotriazle (HOBT). Alernativaly, carbonyl moiety activation of carboxilic acid (II) can be carried out with thionyl chloride or oxalyl chloride in a previous step and the obtained activated compound can be treated with the amine derivative without any purification.

Amines which are suitable to be used in the process of the invention are that of formula R3R4NH where R3 and R4 are independently selected from the group consisting of hydrogen; (C1-C4)alkyl optionally substituted by one to three radicals selected from -OH, a ring of 4-6 members, each member independently selected from the group consisting of C, N, O, S, CH, CH₂, and NH, the ring being saturated, partially unsaturated, or aromatic and optionally substituted by one to three radicals selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, -NRa-C(O)-NRaRb; -(C1-C4)alkyl-C(O)-(C1-C8)alkyl; -(C1-C4)alkyl-COO-(C1-C8)alkyl; -(C1-C4)alkyl-NRaRb; a ring of 3-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and —NRa-C(O)-NRaRb, and a ring of 5-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and -NRa-C(O)-NRaRb. In an alternative embodiment, R3 and R4 together with the N atom to which are attached form a ring of 5-6 members, each member independently selected from C, N, O, S, CH, CH₂, and NH, the ring being saturated or partially unsaturated, optionally substituted with by one to three radicals selected from the group consisting of: halogen; -OH; -(C1-C4)alkyl optionally substituted by -OH; (C1-C4)alkoxyl; -C(O)-(C1-C4)alkyl; -(C1-C4)alkyl-C(O)-(C1-C4)alkyl; -COO-(C1-C4)alkyl; -(C1-C4)alkyl-COO-(C1-C4)alkyl; -NRaRb; -(C1-C4)alkyl-NRaRb; -C(O)-NRaRb; -NRa-C(O)-(C1-C4)alkyl; -NRa-C(O)-NRaRb; and a ring of 3-6 members, each member independently selected from C, N, O, S, CH, CH₂, and NH, the ring being saturated or partially unsaturated. Preferably, amines of formula R3R4NH are selected from the following list,

### List 1

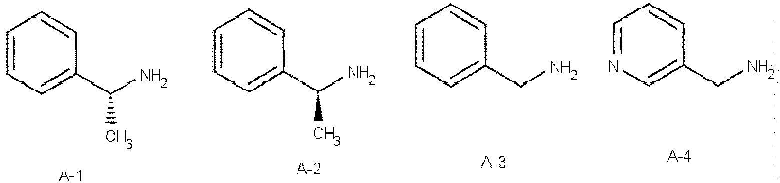

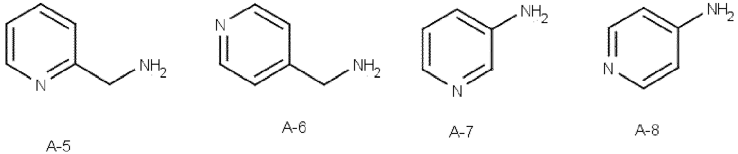

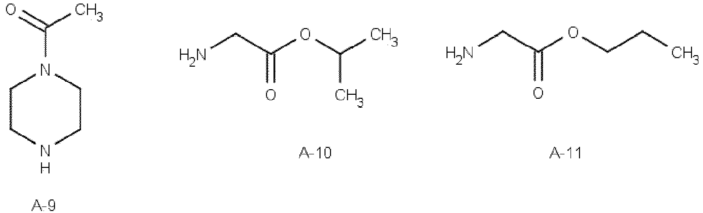

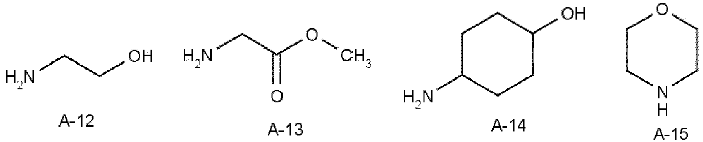

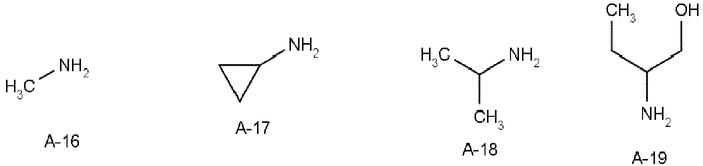

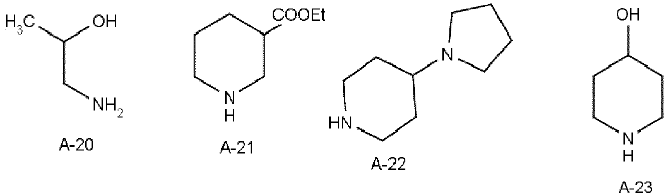

The preparation of pharmaceutically acceptable salts of the compounds of formula (I) can be carried out by methods known in the art. For instance, they can be prepared from the parent compound, which contains a basic moiety, by conventional chemical methods. Generally, such salts are, for example, prepared by the reaction of the free base forms of these compounds with a stoichiometric amount of the appropriate pharmaceutically acceptable acid in water or in an organic solvent or in a mixture of them.

Compounds of formula (II) are new and also form part of the invention. Preferred compounds of formula (II) are those yielding to the preferred compounds of formula (I) mentioned above. The preferred compounds of formula (II) are those where R5 is oxygen and R1 and R2 are independently (C1-C4)alkyl. In an alternative preferred embodiment, R5 is oxygen and R1 and R2 together with the carbon atom to which are attached form a ring of 4-6 members, each member independently selected from the group consisting of C, N, O, S, CH, CH₂, and NH, the ring being saturated or partially unsaturated. More preferably, R1 and R2 are independently selected from the group consisting of (C1-C2)alkyl, or, alternatively R1 and R2 together with the C atom to which are attached, form a ring selected from the group consisting of cyclobutyl, cyclopentyl, cyclohexyl, oxanyl, and tetrahydrothienyl.

Compounds of formula (II) may be prepared by a process comprising the step of reacting a compound of formula (III) 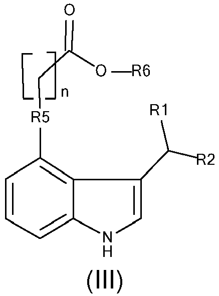 where R6 is a (C1-C4)alkyl and R1, R2, R5, and n are as defined above; with a base. Suitable bases are hydroxides of alkaline or alkaline earth elements, for example LiOH or NaOH, which are commonly used in excess and in an aqueous solvent or in a mixture of aqueous/organic solvent, for instance a water/THF 1:1 mixture.

Compounds of formula (III) are also new and form part of the invention. Preferred compounds of formula (III) are those yielding to the preferred compounds of formula (I) mentioned above. The most preferred compounds of formula (III) are those where R5 is oxygen and R1 and R2 are independently (C1-C4)alkyl. In an alternative further preferred embodiment, R5 is oxygen and R1 and R2 together with the carbon atom to which are attached form a ring of 4-6 members, each member independently selected from the group consisting of C, N, O, S, CH, CH₂, and NH, the ring being saturated or partially unsaturated. More preferably, R1 and R2 are independently selected from the group consisting of (C1-C2)alkyl, or, alternatively R1 and R2 together with the C atom to which are attached, form a ring selected from the group consisting of cyclobutyl, cyclopentyl, cyclohexyl, oxanyl, and tetrahydrothienyl.

These compounds of formula (III) can be prepared by the process shown in Scheme I from the corresponding formyl, amino, or hydroxyl 4-substituted indole.

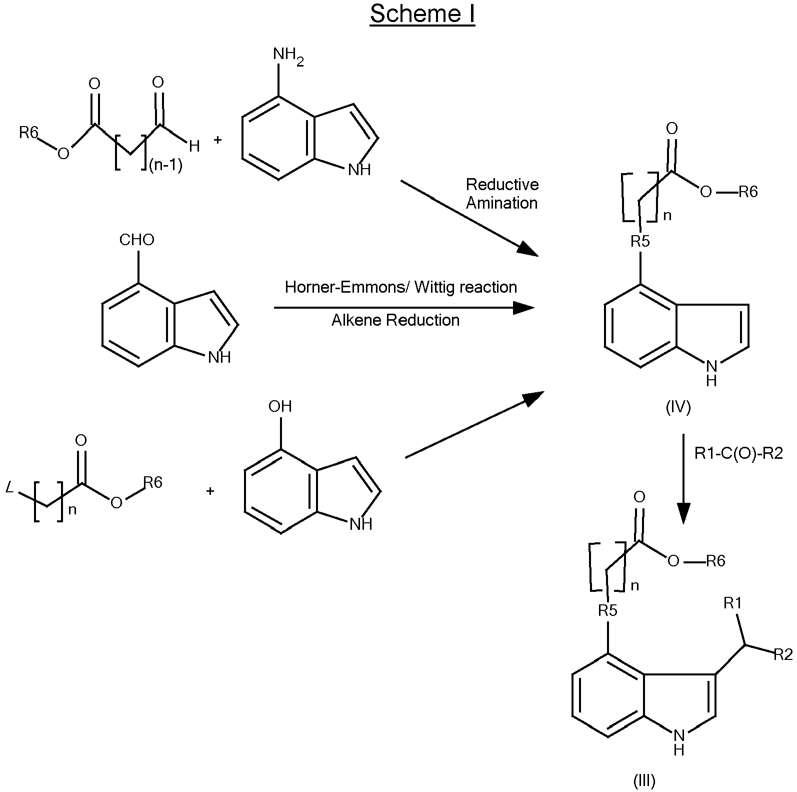

In a first step, the corresponding 4-substituted indole is reacted with an ester moiety containing R6 for yielding the compounds of formula (IV).

When R5 is oxygen, the starting material for preparing compounds of formula (IV) is 4-hydroxy-1H-indole and it can be reacted with the corresponding derivate of formula L-(CH₂)ₙ-COOR6, where L is a leaving group, for example an halogen atom, in particular bromide, in the presence of a base and an organic solvent.

In the cases where R5 is nitrogen, the starting material is 4-amino-1H-indole and it can be reacted with an aldehyde of formula HC(O)-(CH₂)₍ₙ₋₁₎-COOR6 under reductive amination conditions to yield the compounds of formula (IV). Reductive amination can be carried out following known methods in the art, for instance by reacting the 4-amino-1H-indole with the aldehyde derivative at room temperature in the presence of a reducing agent such is sodium cyanoborohydride (NaBH3CN), or sodium triacetoxyborohydride NaBH(OAc)₃ in a - organic solvent as, for example, 1,2-dichloroethane, tetrahydrofuran, or acetonitrile.

In the cases where R5 is a carbon atom, compounds of formula (IV) can be obtained as the product of a two step process comprising a first Horner-Emmons reaction or a Wittig reaction and the subsequent reduction of the alkene formed in the first step. In the Horner-Emmons reaction or the Wittig reaction, the starting material 4-formyl-1H-indole is reacted with a phosphonate ester of formula (EtO)₂-P(O)-(CH₂)ₙ-COOR6 or a triphenyl phosphonium salt containing R6 in the presence of a base as BuLi, NaH, NaOMe in an organic solvent for example THF or diethyleter. The reaction yields the corresponding alkene which is subsequently submitted to a catalytic hydrogenation obtaining reduced compound of formula (IV).

In a second step, compound of formula (IV), obtained by any of the above processes, is reacted with a ketone of formula R1-C(O)-R2, where R1 and R2 are as defined above, to obtain the compound of formula (III). For instance, the compound of formula (IV) can be reacted with the ketone of formula R1-C(O)-R2 in the presence of triethylsilane and trichloride acetic acid in toluene to yield compounds of formula (III).

For example ketones of formula R1-C(O)-R2 can be selected from

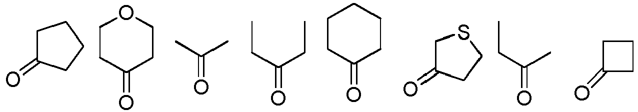

The preparation processes described above can be modified to give enantiopure compounds as well as mixtures of stereoisomers. It is possible to prepare specific stereoisomers or specific mixtures by various processes including the use of stereospecific reagents or by introducing chiral centers into the compounds during its preparation process. In addition, it is possible to separate stereoisomers once the compound has been prepared by standard resolution techniques known to the skilled person.

It has been found that the compounds of formula (I) are useful as medicaments. Formula (I) compounds have showed high activity as inhibitors of bacterial tryptophanyl-tRNA synthetase (WRS), which, as is known in the art, is an evidence of antibacterial activity. WRS belongs to a family of enzymes named aminoacyl-tRNA synthetases (aaRSs) that catalyse the attachment of amino acids to tRNAs. In general aaRSs have been described as promising targets to develop new antibiotics and several aaRS inhibitors have been identified as antibacterial agents, see Tao et al. "Inhibitors of aminoacyl-tRNA synthetases as novel anti-infectives" Expert Opinion on Investigational Drugs 2000, 9 (8), pg. 1767-1775. Different inhibitors of bacterial WRS have been described, some of them being natural compounds such as indolmycin, see Werner et al. "Indolmycin inhibits prokaryotic tryptophanyl-tRNA ligase" European Journal of Biochemistry 1976, 68, pg. 1-3, and chuangxinmycin, see Brown et al. "The antimicrobial natural product chuangxinmycin and some synthetic analogues are potent and selective inhibitors of bacterial tryptophanyl-tRNA synthetase" Bioorganic & Medicinal Chem istry Letters 2002, 12, p 3171-3174.

Therefore, as it is mentioned above, the compounds of formula (I) as defined above for use as a medicament forms part of the invention.

It is also part of the invention the compounds of formula (I) as defined above for use in the prevention and/or treatment of bacterial infections.

This aspect of the invention can be formulated as the use of a compound of formula (I) or its salts, as defined above, for the preparation of a medicament for the treatment of a bacterial infection.

It is also part of the invention a method for the treatment of a subject suffering from a bacterial infection, the method comprising the administration to said subject of a therapeutically effective amount of the compounds of formula (I), together with pharmaceutically acceptable excipients or carriers. In a particular embodiment of the invention the infection is due to Gram negative infection. In another particular embodiment, the infection is due to Gram positive bacteria.

As said above, in a particular embodiment of the invention the compounds of formula (I) are for the use against bacterial infections caused by Gram positive bacteria, for instance *Mycoplasma pneumoniae* and *Staphylococcus aureus.*

*Mycoplasma pneumoniae* is a small bacterium that belongs to the class Mollicutes. Like all other representatives of this class, it does not have a peptidoglycan cell wall that is common for all other firmicute bacteria. *M*. *pneumoniae* is the commonest agent causing atypical pneumonia, a community-acquired pneumonia (CAP) that usually has a prolonged, gradual onset. Lacking a cell wall, these organisms are resistant to the effects of penicillins and other beta-lactam antibiotics, which act by disrupting the bacterial wall. *M. pneumoniae* most often affects younger people, usually between the ages of 5 to 16. Two million people a year in the United States contract this form of pneumonia.

*Staphylococcus aureus* is a gram-positive bacterium that is frequently part of the skin flora, found in the nose and on skin. S. *aureus* can cause a range of illnesses from minor skin infections such as pimples, impetigo, boils (furuncles), cellulitis, folliculitis, carbuncles, scalded skin syndrome, and abscesses or even life-threatening diseases such as pneumonia, meningitis, osteomyelitis, endocarditis, toxic shock syndrome (TSS), chest pain, bacteremia, and sepsis. One of the most common strains is the methicillin-resistant S. *aureus* (MRSA), which is resistant to all beta-lactam antibiotics, like penicillins and cephalosporins. MRSA infections represent a challenge in its treatment especially in hospitals and nursing homes where patients with open wounds, invasive devices and weakened immune systems are exposed to the bacterium and are at greater risk of infection. It is responsible for 30-40% of the total healthcare-associated infections. MRSA causes approximately 100,000 infections worldwide per year, 20% of which are fatal.

All countries have detected the presence of MRSA in hospital systems, but there is also a growing concern in the community acquired MRSA, especially in the US. It can cause from skin- and soft-tissue infections to fatal diseases, including necrotizing pneumonia, severe sepsis and necrotizing fasciitis (associated to 75% lethality). Its worldwide distribution, together with its great ability to generate resistance to existing antibiotics, makes it necessary to develop new chemical entities able to treat MRSA infections and overcome the existing resistance mechanisms.

As said above, in another particular embodiment of the invention the compounds of formula (I) are for the use against bacterial infections caused by Gram negative bacteria.

The compounds of formula (I) can be conveniently administered to a patient in oral unit dosage form. Dosage forms include solid dosage forms like tablets, powders, capsules, sachets, as well as liquid syrups, suspensions and elixirs. The compounds of formula (I) and excipients can be formulated into compositions and dosage forms according to methods known in the art. They can also be used topically as an ingredient in creams, ointments, lotions, gels, foams, soaps, and shampoos. The compounds of formula (I) can also be administered to a patient as an ingredient of injection dosage forms. Injection dosage forms can include liquids for intradermal, intravenous, or subcutaneous injection, solutions for perfusion, powder for reconstitution of liquid injections, and pre-filled syringes.

The compounds of formula (I) can be used either alone or in combination with other therapeutic agents, for example antifungals, and other antibiotics.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Reference Example: General preparation of compounds of formula (IV) when R5 is O

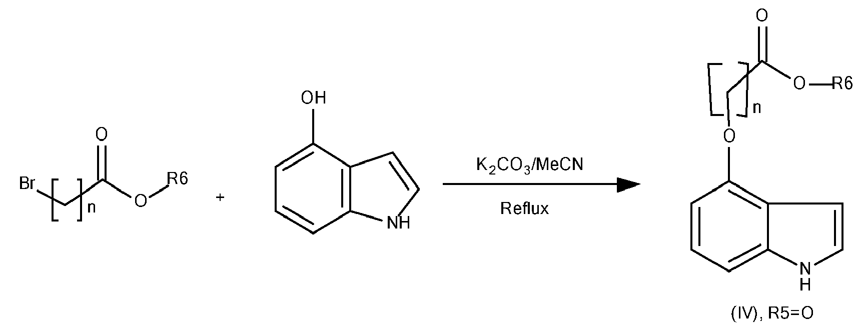

The corresponding bromoderivate (1.1 eq) and K₂CO₃ (1.5 eq) were added to a solution of 1H-indol-4-ol (1 eq) in MeCN (1 M). The resulting mixture was refluxed for 4 h. MeCN was evaporated and the residue was diluted with ethyl acetate (EtOAc) and washed with water. The organic layer was dried and evaporated to afford the resulting compounds of formula (IV). The products were separated by HPLC method A, B, or C and its structure was determined by Mass Spectrometry:

### Method A

Column; Waters X-Bridge-C18 50 mm x 4.6 mm x 3.5 um
Mobile Phase: A:ACN+0.05%TFA B:Water+0.05%TFA
Gradient Program: A from 5 to 100 for 5.0 min and hold for 1.0 min
Flow Rate: 2.0 ml/min

### Method B

Column;Phenomenex Kinetex C18 50 mm x 4.6 mm x 3.5 um
Mobile Phase: A:ACN+0.2%HCOOH B:Water+0.2%HCOOH
Gradient Program: A from 5 to 90 for 3.0 min and hold for 1.0 min
Flow Rate: 1.0 ml/min

### Method C

Column: X Bridge C18, 50 mm x 4.6 mm x 3.5 um;
Mobile Phase: A: 0.05%TFA in ACN, B: 0.05%TFA in water
Flow rate: 2.0 ml/min;
Gradient: From 5% ACN to 100% ACN for 1.6 min and hold for 0.7 min;
Column Temp: 50 °C

The following compounds of formula (IV) were prepared according the previous process using the corresponding bromo-ester derivatives.

### (IV)-1 Ethyl 2-((1H-indol-4-yl)oxy)acetate

Compound of formula (IV) with R5=O, n=1 and R6 = -CH₂-CH₃
Analytical Method A; Retention time 2.610 min
MS (m/z) 220 (M+1)

### (IV)-2 Ethyl 4-((1H-indol-4-yl)oxy)butanoate

Compound of formula (IV) with R5=O, n=3 and R6 = -CH₂-CH₃
Analytical Method A; Retention time 3.133 min
MS (m/z) 248 (M+1)

### (IV)-3 Methyl 2-((1H-indol-4-yl)oxy)acetate

Compound of formula (IV) with R5=O, n=1 and R6 = -CH₃
See, Zhou N. et al. "3,4-Disubstituted indole acylsulfonamides: a novel series of potent and selective human EP3 receptor antagonists" Bioorganic & Medicinal Chemistry Letters, 2009, 19, pg. 123-126.

### Example 1. Preparation of compounds of formula (III) when R5 is O

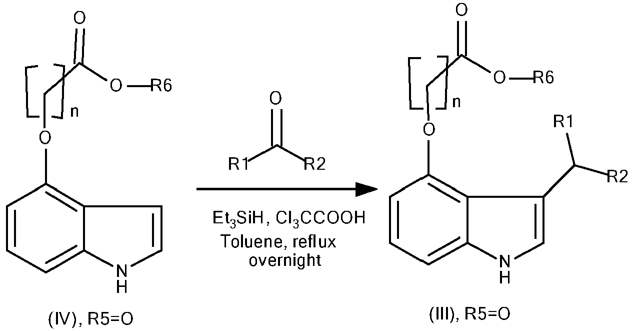

### General preparation:

Et₃SiH (3 eq), ketone of formula R1-C(O)-R2 (1.1 eq) and Cl₃CCOOH (1.5 eq) were added to a solution of the compounds of formula (IV) (1 eq), obtained in the previous step, in of toluene (c=0.45 M). The mixture was heated at 70 °C overnight and poured into ice-water. The organic layer was washed with saturated NaHCO₃ aqueous solution, dried and evaporated to afford crude product. The residue was purified by a flash silica gel column to afford compounds of formula (III). The following compounds of formula (III) were prepared according the previous process using the corresponding ketones.

### (III)-1 Ethyl 2-((3-(tetrahydro-2H-pyran-4-yl)-1H-indol-4-yl)oxy)acetate

Starting material: Compound (IV)-1
Analytical Method A; Retention time 3.267 min
MS (m/z) 304 (M+1)

### (III)-2 Ethyl 2-((3-(pentan-3-yl)-1H-indol-4-yl)oxy)acetate

Starting material: Compound (IV)-1
Analytical Method A; Retention time 3.821 min
MS (m/z) 290 (M+1)

### (III)-3 Ethyl 2-((3-(tetrahydrothiophen-3-yl)-1H-indol-4-yl)oxy)acetate

Starting material: Compound (IV)-1
Analytical Method C; Retention time 1.909 min
MS (m/z) 306 (M+1)

### (III)-4 Ethyl 2-((3-cyclobutyl-1H-indol-4-yl)oxy)acetate

Starting material: Compound (IV)-1
Analytical Method A; Retention time 3.665 min
MS (m/z) 274 (M+1)

### (III)-5 Ethyl 2-((3-cyclopentyl-1H-indol-4-yl)oxy)acetate

Starting material: Compound (IV)-1
Analytical Method A; Retention time 3.683 min
MS (m/z) 288 (M+1)

### (III)-6 Ethyl 2-((3-isopropyl-1H-indol-4-yl)oxy)acetate

Starting material: Compound (IV)-1
Analytical Method C; Retention time 1.927 min
MS (m/z) 262 (M+1)

### (III)-7 Ethyl 2-((3-cyclohexyl-1H-indol-4-yl)oxy)acetate

Starting material: Compound (IV)-1
Analytical Method A; Retention time 4.331 min
MS (m/z) 302 (M+1)

### (III)-8 Methyl 2-((3-(sec-butyl)-1H-indol-4-yl)oxy)acetate

Starting material: Compound (IV)-3
¹H NMR (500 MHz, Acetone) δ 9.99 (br, 1H), 7.16 - 6.89 (m, 3H), 6.40 (d, J = 7.6 Hz, 1H), 4.82 (s, 2H), 3.79 (s, 3H), 3.44 (h, J = 6.9 Hz, 1H), 1.94 - 1.80 (m, 1H), 1.64 - 1.51 (m, 1H), 1.32 (d, J = 6.9 Hz, 3H), 0.95 (t, J = 7.4 Hz, 3H).

### (III)-9 Ethyl 4-((3-cyclobutyl-1H-indol-4-yl)oxy)butanoate

Starting material: Compound (IV)-2.
Analytical Method A; Retention time 4.050 min
MS (m/z) 302 (M+1)

### (III)-10 Ethyl 4-((3-cyclopentyl-1H-indol-4-yl)oxy)butanoate

Starting material: Compound (IV)-2
Analytical Method A; Retention time 4.000 min
MS (m/z) 316 (M+1)

### (III)-11 Ethyl 4-((3-isopropyl-1H-indol-4-yl)oxy)butanoate

Starting material: Compound (IV)-2
Analytical Method A; Retention time 3.755 min
MS (m/z) 290 (M+1)

### Example 2. Preparation of compounds of formula (II) when R5 is O

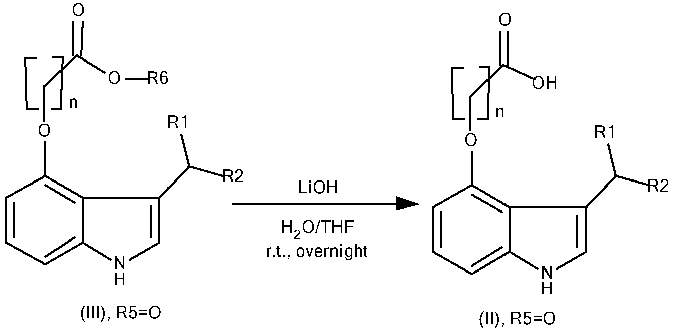

### General preparation:

LiOH (6 M) (2.6eq.) was added to a solution of (II) (1eq.), obtained according to the previous step, in THF (c=0.46 M) and the resulting solution was stirred overnight at room temperature (r.t.). After solvent evaporation, the residue was diluted with water and acidified with aqueous HCl until pH=1-2. The organic layer was extracted with dichloromethane (DCM). The combined organic layer was dried and evaporated to afford the compounds of formula (II). The following compounds of formula (II) were prepared according the previous process.

### (II)-1 2-((3-(tetrahydro-2H-pyran-4-yl)-1H-indol-4-yl)oxy)acetic acid

Starting material: Compound (III)-1
Analytical Method A; Retention time 2.414 min
MS (m/z) 276 (M+1)

### (II)-2 2-((3-(pentan-3-yl)-1H-indol-4-yl)oxy)acetic acid

Starting material: Compound (III)-2
Analytical Method A; Retention time 2.991 min
MS (m/z) 262 (M+1)

### (II)-3 2-((3-(tetrahydrothiophen-3-yl)-1H-indol-4-yl)oxy)acetic acid

Starting material: Compound (III)-3
Analytical Method A; Retention time 2.594 min
MS (m/z) 278 (M+1)

### (II)-4 2-((3-cyclobutyl-1H-indol-4-yl)oxy)acetic acid

Starting material: Compound (III)-4
Analytical Method A; Retention time 2.667 min
MS (m/z) 246 (M+1)

### (II)-5 2-((3-cyclopentyl-1H-indol-4-yl)oxy)acetic acid

Starting material: Compound (III)-5
Analytical Method A; Retention time 2.838 min
MS (m/z) 260 (M+1)

### (II)-6 2-((3-isopropyl-1H-indol-4-yl)oxy)acetic acid

Starting material: Compound (III)-6
Analytical Method C; Retention time 1.666 min
MS (m/z) 234 (M+1)

### (II)-7 2-((3-cyclohexyl-1H-indol-4-yl)oxy)acetic acid

Starting material: Compound (III)-7
Analytical Method A; Retention time 3.407 min
MS (m/z) 274 (M+1)

### (II)-8 2-((3-(sec-butyl)-1H-indol-4-yl)oxy)acetic acid

Starting material: Compound (III)-8
¹H NMR (500 MHz, Acetone) δ 10.00 (br, 1H), 7.08 - 6.87 (m, 3H), 6.42 (d, J = 7.5 Hz, 1H), 4.79 (s, 2H), 3.45 (m, 1H), 3.18 (br, 1H), 1.95 - 1.79 (m, 1H), 1.95 - 1.75 (m, 1H), 1.62 - 1.49 (m, 1H), 1.31 (d, J = 6.9 Hz, 3H), 0.93 (t, J = 7.4 Hz, 3H).

### (II)-9 4-((3-cyclobutyl-1H-indol-4-yl)oxy)butanoic acid

Starting material: Compound (III)-9
Analytical Method A; Retention time 3.212 min
MS (m/z) 274 (M+1)

### (II)-10 4-((3-cyclopentyl-1H-indol-4-yl)oxy)butanoic acid

Starting material: Compound (III)-10
Analytical Method A; Retention time 3.809 min
MS (m/z) 288 (M+1)

### (II)-11 4-((3-isopropyl-1H-indol-4-yl)oxy)butanoic acid

Starting material: Compound (III)-11
Analytical Method A; Retention time 2.835 min
MS (m/z) 262 (M+1)

### Example 3. Preparation of compounds of formula (I) when R5 is O

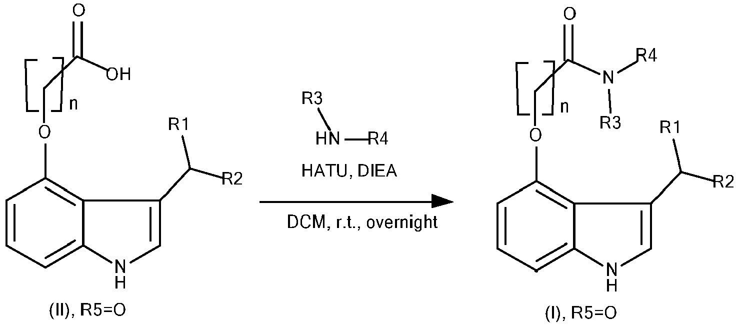

### General preparation:

N,N-Diisopropylethylamine (DIEA) (1.5 eq) and O-(7-Azabenzotriazol-1-yl)-N, N, N', N'-tetramethyluronium hexafluorophosphate (HATU) (1.1 eq) were added to a solution of (II) (1 eq), obtained according to the previous step, in dichloromethane (DCM) (c=0.18M). The corresponding amine (1.2 eq) was also added to the solution and the mixture was stirred overnight at room temperature. The obtained compound (I) was purified by HPLC.

The amines of formula HNR3R4 are selected from that of the List 1 above.

The compounds of formula (I) listed in the following Table I were prepared according the previous process using the corresponding amine compound.

**Table I**

| Compound | Starting Material | Amine | Retention Time (min) Analytical Method | MS (m/z) |
|---|---|---|---|---|
| (I)-1 | (II)-3 | A-4 | 2.31; A | 368 (M+1) |
| (I)-2 | (II)-3 | A-1 | 3.77; A | 381 (M+1) |
| (I)-3 | (II)-3 | A-8 | 2.08; A | 354 (M+1) |
| (I)-4 | (II)-3 | A-7 | 2.05; A | 354 (M+1) |
| (I)-5 | (II)-3 | A-5 | 2.38; A | 368 (M+1) |
| (I)-6 | (II)-3 | A-3 | 3.61; A | 367 (M+1) |
| (I)-7 | (II)-3 | A-6 | 2.27; A | 368 (M+1) |
| (I)-8 | (II)-3 | A-2 | 3.76; A | 381 (M+1) |
| (I)-9 | (II)-3 | A-11 | 3.54; A | 377 (M+1) |
| (I)-10 | (II)-3 | A-17 | 2.78; A | 317 (M+1) |
| (I)-11 | (II)-3 | A-18 | 3.52; A | 319 (M+1) |
| (I)-12 | (II)-3 | A-19 | 2.48; A | 349 (M+1) |
| (I)-13 | (II)-3 | A-10 | 3.33; A | 377 (M+1) |
| (I)-14 | (II)-3 | A-14 | 2.43; A | 375 (M+1) |
| (I)-15 | (II)-3 | A-20 | 2.67; A | 335 (M+1) |
| (I)-16 | (II)-3 | A-13 | 2.95; A | 349 (M+1) |
| (I)-17 | (II)-2 | A-6 | 2.37; A | 352 (M+1) |
| (I)-18 | (II)-2 | A-4 | 2.37; A | 352 (M+1) |
| (I)-19 | (II)-2 | A-7 | 2.54; A | 338 (M+1) |
| (I)-20 | (II)-2 | A-5 | 2.46; A | 352 (M+1) |
| (I)-21 | (II)-2 | A-9 | 2.75; A | 372 (M+1) |
| (I)-22 | (II)-2 | A-13 | 3.07; A | 333 (M+1) |
| (I)-23 | (II)-2 | A-15 | 3.06; A | 331 (M+1) |
| (I)-24 | (II)-2 | A-11 | 3.5; A | 361 (M+1) |
| (I)-25 | (II)-2 | A-19 | 3.08; A | 333 (M+1) |
| (I)-26 | (II)-2 | A-16 | 2.97; A | 275 (M+1) |
| (I)-27 | (II)-2 | A-17 | 3.34; A | 301 (M+1) |
| (I)-28 | (II)-2 | A-12 | 2.6; A | 305 (M+1) |
| (I)-29 | (II)-2 | A-14 | 2.8; A | 359 (M+1) |
| (I)-30 | (II)-2 | A-23 | 2.75; A | 345 (M+1) |
| (I)-31 | (II)-1 | A-1 | 3.26; A | 379 (M+1) |
| (I)-32 | (II)-1 | A-2 | 3.26; A | 379 (M+1) |
| (I)-33 | (II)-1 | A-3 | 3.13; A | 365 (M+1) |
| (I)-34 | (II)-1 | A-5 | 1.99; A | 366 (M+1) |
| (I)-35 | (II)-1 | A-4 | 1.92; A | 366 (M+1) |
| (I)-36 | (II)-1 | A-6 | 1.91; A | 366 (M+1) |
| (I)-37 | (II)-1 | A-7 | 2.06; A | 352 (M+1) |
| (I)-38 | (II)-1 | A-10 | 2.96; A | 375 (M+1) |
| (I)-39 | (II)-1 | A-15 | 2.4; A | 345 (M+1) |
| (I)-40 | (II)-1 | A-13 | 2.47; A | 347 (M+1) |
| (I)-41 | (II)-4 | A-6 | 2.08; A | 336 (M+1) |
| (I)-42 | (II)-4 | A-5 | 2.18; A | 336 (M+1) |
| (I)-43 | (II)-4 | A-9 | 2.41; A | 356 (M+1) |
| (I)-44 | (II)-7 | A-5 | 2.84; A | 364 (M+1) |
| (I)-45 | (II)-7 | A-12 | 2.99; A | 317 (M+1) |
| (I)-46 | (II)-7 | A-13 | 3.48; A | 345 (M+1) |
| (I)-47 | (II)-5 | A-4 | 2.23; A | 350 (M+1) |
| (I)-48 | (II)-5 | A-9 | 2.59; A | 370 (M+1) |
| (I)-49 | (II)-5 | A-12 | 2.46; A | 303 (M+1) |
| (I)-50 | (II)-5 | A-15 | 2.89; A | 329 (M+1) |
| (I)-51 | (II)-5 | A-17 | 3.08; A | 299 (M+1) |
| (I)-52 | (II)-5 | A-16 | 2.83; A | 273 (M+1) |
| (I)-53 | (II)-5 | A-3 | 3.51; A | 349 (M+1) |
| (I)-54 | (II)-5 | A-14 | 2.68; A | 357 (M+1) |
| (I)-55 | (II)-5 | A-19 | 2.82; A | 331 (M+1) |
| (I)-56 | (II)-5 | A-11 | 3.38; A | 359 (M+1) |
| (I)-57 | (II)-5 | A-10 | 3.36; A | 359 (M+1) |
| (I)-58 | (II)-6 | A-6 | 1.99; A | 324 (M+1) |
| (I)-59 | (II)-6 | A-8 | 2.15; A | 310 (M+1) |
| (I)-60 | (II)-6 | A-4 | 1.98; A | 324 (M+1) |
| (I)-61 | (II)-6 | A-7 | 2.14; A | 310 (M+1) |
| (I)-62 | (II)-6 | A-2 | 3.45; A | 337 (M+1) |
| (I)-63 | (II)-6 | A-5 | 2.09; A | 324 (M+1) |
| (I)-64 | (II)-6 | A-10 | 3.12; A | 333 (M+1) |
| (I)-65 | (II)-6 | A-17 | 2.82; A | 273 (M+1) |
| (I)-66 | (II)-6 | A-15 | 2.6; A | 303 (M+1) |
| (I)-67 | (II)-6 | A-13 | 2.67; A | 305 (M+1) |
| (I)-68 | (II)-8 | A-21 | 2.289; B | 387 (M+1) |
| (I)-69 | (II)-9 | A-17 | 3.381; A | 313 (M+1) |
| (I)-70 | (II)-10 | A-15 | 3.407; A | 343 (M+1) |
| (I)-71 | (II)-11 | A-23 | 2.877; A | 345 (M+1) |
| (I)-72 | (II)-9 | A-23 | 2.945; A | 357 (M+1) |
| (I)-73 | (II)-10 | A-22 | 3.215; A | 424 (M+1) |

### Example 4. Biological Activity

The compounds of formula (I) have been tested for antibacterial activity in vitro as aaRS inhibitors following the following process.

### Protein expression and purification

*Escherichia coli* M15 cells transformed with plasmid pQE-60 containing the open-reading frame sequence of WRS from *Mycoplasma pneumoniae* (MpnWRS) were induced with 1 mM IPTG (Isopropyl β-D-1-thiogalactopyranoside) for 3 h at 37°C. Bacterial cells were harvested and lysed with 20 mM NaH₂PO₄ (pH 8.0), 200 mM NaCl, 10 mM imidazole and protease inhibitor cocktail (Roche). Tryptophanyl-tRNA synthetase from *M. pneumoniae* was purified by nickel affinity standard cromatography. Protein concentration was determined by Bradford method (Bio-Rad).

### In vitro tRNA transcription

*Mycoplasma pneumoniae* tRNA^{TrP} was transcribed in vitro for 4 h at 37°C using T7 RNA polymerase (New England Biolabs). Transcription reaction contained 40 mM Tris-HCl (pH 8.0), 20 mM MgCl₂, 1 mM spermidine, 5 mM DTT, 0.01% Triton X-100, 4 mM GTP, 4mM ATP, 4 mM UTP, 4 mM CTP, 16 mM GMP, 250 units T7 RNA polymerase and 150 µg BstNI digested plasmid. Once finished, the reaction was applied on a 6% polyacrylamide-8 M urea denaturing gel to purify the transcribed tRNA^{TrP} and discard any impurities. Purified tRNA was quantified with Nanodrop 2000 (Thermo Scientific).

### Determination of IC50

The aminoacylation reaction catalyzed by aminoacyl-tRNA synthetases (aaRS) takes place in two steps. In the first step, aaRS activate its cognate amino acid with ATP; and in the second step the activated amino acid is loaded to its corresponding tRNA. This reaction can be summarized as follows:
[1] ARS + aa + ATP = ARS-aa-AMP + PPi
[2] ARS-aa-AMP + tRNA = aa-tRNA + AMP + ARS
(ARS, Aminoacyl-tRNA synthetase; aa, amino acid; ARS-aa-AMP, enzyme-bound to aminoacyl-adenylate; aa-tRNA, aminoacyl-tRNA)

IC50 determination for compounds of formula (I) was performed using the purified tryptophanyl-tRNA synthetase from *Mycoplasma pneumoniae* (MpnWRS). The activity of this enzyme was monitored by measuring the ATP consumption rate with the commercial kit Kinase RR (BioThema AB, Sweden) in the presence of serial dilutions of inhibitor following manufacturer instructions. Indolmycin, a well known inhibitor of tryptophanyl-tRNA synthetase, was used as a positive control of the assay. IC50 was calculated with ChemBioOffice Enterprise software (PerkinElmer).

The following table shows the aaRS inhibitory activity of the compounds of formula (I), categorised as compounds which showed IC50 below 20 µM (shown as <20) and compounds which showed an IC50 between 20 and 50 µM (shown as 20-50).

| **Compound** | **IC50 pM BT MpnWRS** | | **Compound** | **IC50 pM BT MpnWRS** |
|---|---|---|---|---|
| (I)-1 | <20 | | (I)-59 | <20 |
| (I)-2 | <20 | | (I)-60 | <20 |
| (I)-3 | <20 | | (I)-61 | <20 |
| (I)-4 | <20 | | (I)-62 | <20 |
| (I)-5 | <20 | | (I)-63 | <20 |
| (I)-6 | <20 | | (I)-64 | <20 |
| (I)-7 | <20 | | (I)-10 | 20-50 |
| (I)-8 | <20 | | (I)-11 | 20-50 |
| (I)-9 | <20 | | (I)-12 | 20-50 |
| (I)-17 | <20 | | (I)-13 | 20-50 |
| (I)-18 | <20 | | (I)-14 | 20-50 |
| (I)-19 | <20 | | (I)-15 | 20-50 |
| (I)-20 | <20 | | (I)-16 | 20-50 |
| (I)-21 | <20 | | (I)-24 | 20-50 |
| (I)-22 | <20 | | (I)-25 | 20-50 |
| (I)-23 | <20 | | (I)-26 | 20-50 |
| (I)-31 | <20 | | (I)-27 | 20-50 |
| (I)-32 | <20 | | (I)-28 | 20-50 |
| (I)-33 | <20 | | (I)-29 | 20-50 |
| (I)-34 | <20 | | (I)-30 | 20-50 |
| (I)-35 | <20 | | (I)-40 | 20-50 |
| (I)-36 | <20 | | (I)-43 | 20-50 |
| (I)-37 | <20 | | (I)-46 | 20-50 |
| (I)-38 | <20 | | (I)-51 | 20-50 |
| (I)-39 | <20 | | (I)-52 | 20-50 |
| (I)-41 | <20 | | (I)-53 | 20-50 |
| (I)-42 | <20 | | (I)-54 | 20-50 |
| (I)-44 | <20 | | (I)-55 | 20-50 |
| (I)-45 | <20 | | (I)-56 | 20-50 |
| (I)-47 | <20 | | (I)-57 | 20-50 |
| (I)-48 | <20 | | (I)-65 | 20-50 |
| (I)-49 | <20 | | (I)-66 | 20-50 |
| (I)-50 | <20 | | (I)-67 | 20-50 |
| (I)-58 | <20 | | (I)-68 | 20-50 |

### REFERENCES CITED IN THE APPLICATION

WO1998057931
W02006044000
Pereira et al. "Synthesis and antimicrobial activities of five membered ring heterocycles coupled to indole moieties", The Journal of Antibiotics, 1996, Vol. 49, n. 4, pp 380-385
Jasbir Singh et al. "Structure-Activity Relationship Studies Leading to the Identification of (2E)-3-[I-[(2,4-Dichlorophenyl)methyl]-5-fluoro-3-methyl-1H-indol-7-yl]-N-[(4,5-dichloro-2-thienyl)sulfonyl]-2-propenamide (DG-041), a Potent and Selective Prostanoid EP3 Receptor Antagonist, as a Novel Antiplatelet Agent That Does Not Prolong Bleeding" Journal of Medicinal Chemistry 2010, No. 53, pgs. 18-36.
Brown et al. "The antimicrobial natural product chuangxinmycin and some synthetic analogues are potent and selective inhibitors of bacterial tryptophanyl-tRNA synthetase" Bioorganic & Medicinal Chemistry Letters 2002, 12, p 3171-3174.
Tao et al. "Inhibitors of aminoacyl-tRNA synthetases as novel anti-infectives" Expert Opinion on Investigational Drugs 2000, 9 (8), p 1767-1775.
Werner et al. "Indolmycin inhibits prokaryotic tryptophanyl-tRNA ligase" European Journal of Biochemistry 1976, 68, p 1-3.
Zhou N. et al. "3,4-Disubstituted indole acylsulfonamides: a novel series of potent and selective human EP3 receptor antagonists" Bioorganic & Medicinal Chemistry Letters 19 (2009) 123―126.

## Claims

1. A compound of formula (I) or a pharmaceutically acceptable salt thereof, or a stereoisomer thereof or a mixture of stereoisomers, 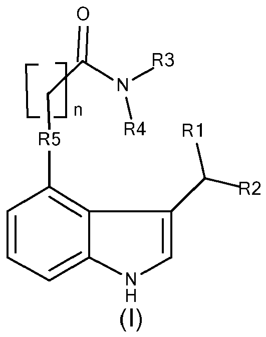 wherein,
R1 and R2 are independently (C1-C4)alkyl, or alternatively, R1 and R2 together with the C atom to which are attached form a ring of 4-7 members, each member independently selected from the group consisting of C, N, O, S, CH, CH₂, and NH, the ring being saturated or partially unsaturated and optionally substituted by one to three radicals selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, -NRa-C(O)-NRaRb, and benzyl;
R3 and R4 are independently selected from the group consisting of hydrogen; (C1-C4)alkyl optionally substituted by one to three radicals selected from -OH, a ring of 4-6 members, each member independently selected from the group consisting of C, N, O, S, CH, CH₂, and NH, the ring being saturated, partially unsaturated, or aromatic and optionally substituted by one to three radicals selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, -NRa-C(O)-NRaRb; -(C1-C4)alkyl-C(O)-(C1-C8)alkyl; -(C1-C4)alkyl-COO-(C1-C8)alkyl; -(C1-C4)alkyl-NRaRb; a ring of 3-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and -NRa-C(O)-NRaRb, and a ring of 5-6 members, each member independently selected from C, N, O, S, CH, CH₂, and NH, the ring being saturated, partially unsaturated, or aromatic, and optionally substituted by one to three radicals independently selected from the group consisting of: halogen, (C1-C4)alkyl, (C1-C4)alkoxyl, -OH, -NRaRb, -(C1-C4)alkyl-NRaRb, -C(O)-NRaRb, -NRa-C(O)-(C1-C4)alkyl, and -NRa-C(O)-NRaRb; or alternatively,
R3 and R4 together with the N atom to which are attached form a ring of 5-6 members, each member independently selected from C, N, O, S, CH, CH₂, and NH, the ring being saturated or partially unsaturated, optionally substituted with by one to three radicals selected from the group consisting of: halogen; -OH; -(C1-C4)alkyl optionally substituted by ―OH; (C1-C4)alkoxyl; -C(O)-(C1-C4)alkyl; -(C1-C4)alkyl-C(O)-(C1-C4)alkyl; -COO-(C1-C4)alkyl; -(C1-C4)alkyl-COO-(C1-C4)alkyl; -NRaRb; -(C1-C4)alkyl-NRaRb; -C(O)-NRaRb; -NRa-C(O)-(C1-C4)alkyl; -NRa-C(O)-NRaRb; and a ring of 3-6 members, each member independently selected from C, N, O, S, CH, CH₂, and NH, the ring being saturated or partially unsaturated.
R5 is an atom selected from the group consisting of N, C, and O;
n is an integer from 1 to 3; and
Ra and Rb are independently selected from the group consisting of H and (C1-C4)alkyl.

2. The compound according to claim 1, wherein R5 is O and n is 1.

3. The compound according to any of the claims 1-2, wherein R1 and R2 are independently (C1-C4)alkyl, or alternatively, R1 and R2 together with the C atom to which are attached form a ring of 4-6 members, each member independently selected from the group consisting of C, N, O, S, CH, CH₂, and NH, the ring being saturated or partially unsaturated.

4. The compound according to claim 3, wherein
R1 and R2 are independently selected from the group consisting of (C1-C2)alkyl;
R1 and R2 together with the C atom to which are attached, form a ring selected from the group consisting of cyclobutyl, cyclopentyl, cyclohexyl, oxanyl, and tetrahydrothienyl.

5. The compound according to any of the claims 1-4, wherein
R3 and R4 are independently selected from the group consisting of hydrogen; (C1-C4)alkyl optionally substituted by one to three radicals selected from -OH, a ring of 5-6 members, each member independently selected from the group consisting of C, N, O, S, CH, CH₂, and NH, the ring being saturated, partially unsaturated, or aromatic; -CH₂-COO-(C1-C4)alkyl; and a ring of 3-6 members, each member independently selected from C, N, O, CH, CH₂, and NH, the ring being saturated, partially unsaturated, or aromatic, optionally substituted by one to three radicals selected from the group consisting of: -OH, and -(C1-C4)alkyl;
R3 and R4 together, with the N atom within, form a ring of 5-6 members, each member independently selected from C, N, O, CH, CH₂, and NH, the ring being saturated or partially unsaturated, optionally substituted by one to three radicals selected from the group consisting of: -OH; -(C1-C4)alkyl optionally substituted by ―OH; -C(O)-(C1-C4)alkyl; -COO-(C1-C4)alkyl; and a ring of 5-6 members, each member independently selected from C, N, O, S, CH, CH₂, and NH, the ring being saturated.

6. The compound according to any of the claims 1-5, wherein
R3 and R4 are independently selected from the group consisting of hydrogen; (C1-C4)alkyl optionally substituted by one to three radicals selected from -OH, phenyl, and pyridinyl; -CH₂-COO-(C1-C4)alkyl; pyridinyl; cyclopropyl, and cyclohexyl, optionally substituted with ―OH;
R3 and R4 together with the N atom to which are attached form a ring selected from the group consisting of piperazine, morpholine, and piperidine; optionally substituted by -C(O)-(C1-C4)alkyl, -COO-(C1-C4)alkyl, and pyrrolidinyl.

7. The compound according to any of the claims 1-6, wherein
R3 and R4 are independently selected from the group consisting of hydrogen; (C1-C2)alkyl optionally being substituted by one to three radicals selected from -OH, phenyl and pyridinyl; -CH₂-COO-(C1-C3)alkyl; and pyridinyl;
R3 and R4 together with the N atom to which are attached, form a group selected from the group consisting of piperazine, and morpholine; optionally substituted by -C(O)-(C1-C2)alkyl.

8. A process for the preparation of a compound of formula (I) as defined in any of the claims 1-7, comprising the step of reacting a compound of formula (II), 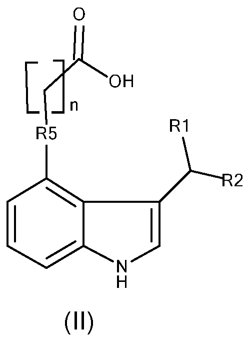 wherein R1, R2, R5, and n are as defined in any of the claims 1-7; with an amine of formula R3R4NH, wherein R3 and R4 are as defined in any of the claims 1-7.

9. The process according to claim 8, further comprising a previous step of reacting a compound of formula (III), 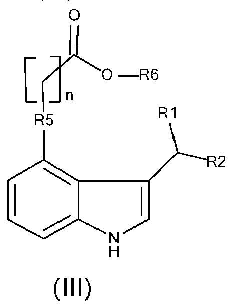 wherein R6 is a (C1-C4)alkyl and R1, R2, R5, and n are as defined in any of the claims 1-7;
with a base to render a compound of formula (II).

10. A compound of formula (II) 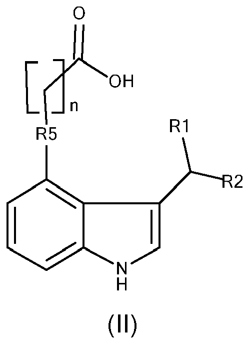 wherein R1, R2, R5, and n are as defined in any of the claims 1-7.

11. A compound of formula (III), 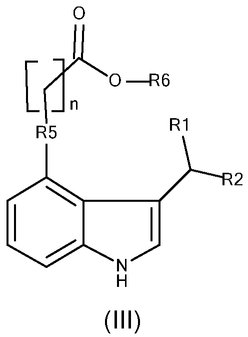 wherein R6 is a (C1-C4)alkyl, and R1, R2, R5, and n are as defined in any of the claims 1-7.

12. A compound of formula (I) as defined in any of the claims 1-7 for use as a medicament.

13. The compound according to claim 12, for use as an antibacterial agent.

14. A pharmaceutical composition comprising a therapeutically effective amount of the compound as defined in any of the claims 1-7, together with adequate amounts of pharmaceutical excipients or carriers.
